# EUROPEAN PATENT APPLICATION

(11) **EP 3 156 097 A1**
(43) Date of publication of application: **19.04.2017**
(21) Application number: 15806520.1
(22) Date of filing: 02.06.2015
(51) Int. Cl.: A61M 37/00

(54) **MICRONEEDLE SHEET AND DERMAL ADMINISTRATION PLASTER**

(30) Priority: 10.06.2014 JP 2014119370
(71) Applicant: Nissha Printing Co., Ltd., Kyoto-shi, Kyoto 604-8551 (JP)
(72) Inventor: NAGAI, Hiroyuki, Kyoto-shi Kyoto 604-8551 (JP); SUETOMI, Yoshiko, Kyoto-shi Kyoto 604-8551 (JP); YOSHIZUKA, Saori, Kyoto-shi Kyoto 604-8551 (JP)
(74) Representative: Kastel, Stefan
(86) International application number: PCT/JP2015/065842
(87) International publication number: WO 2015/190344

(57) **Abstract**

Provided are a transdermal patch and a microneedle sheet, which can efficiently introduce a target substance to the stratum corneum and prevent a reduction in the protection capability of the stratum corneum. A microneedle sheet (20) comprises a sheet-shaped base material (21) and a plurality of microneedles (50) on the sheet-shaped base material (21) making contact with a stratum corneum (110) of a skin (100). Each of the plurality of microneedles (50) includes a skin-stretching part (51) projecting from the sheet-shaped base material (21), the skin-stretching part having a tip surface (51 a) configured to stretch the stratum corneum (110) by pressing against the surface of the stratum corneum (110) of the skin (100) without sticking into the surface of the stratum corneum (110). A needlelike-projection part (52) is formed on the tip surface (51a) of the skin-stretching part (51) having a shape such that the needlelike-projection part (52) sticks into the surface of the stratum corneum and a peak part (52a) of the needlelike-projection part (52) stays inside the stratum corneum (110).

## Description

### TECHNICAL FIELD

The present invention relates to a microneedle sheet and a transdermal patch for introducing a target substance to the stratum corneum of the skin using a plurality of microneedles.

### BACKGROUND ART

Conventionally, one means of noninvasively administering a drug or the like via the body surface of an organism, such as the skin or a mucous membrane, is to perform transdermal administration via a transdermal patch. Furthermore, to efficiently absorb a drug or the like from a transdermal patch into the body, a preparation called a microneedle sheet or a microneedle patch is being developed whereon the drug is adsorbed by minute needles having a high aspect ratio, which are referred to as so-called microneedles, and those minute needles are disposed in an array on a sheet. For example, Patent Citation 1 (Japanese Patent No. 5472771) discloses a technique in which a drug is provided on microneedles, which perforate the stratum corneum, thereby introducing the medicine as far as an inner part of the skin at a part that is deeper than the stratum corneum.

### CITATION LIST

### PATENT CITATIONS

Patent Citation 1: Japanese Patent No. 5472771

### SUMMARY OF INVENTION

### TECHNICAL PROBLEM

Nevertheless, when it is desired to introduce a target substance, such as a medicine, to the stratum corneum or to a deep part of the skin by passing through the stratum corneum, if the stratum corneum is perforated by the microneedles, then through holes are adversely formed that directly pass through from the stratum corneum to, for example, the stratum granulosum below the stratum corneum. Thus, if through holes are formed in the stratum corneum, then the protection capability-i.e., protection of the dermis, the hypodermis, and the like from microbes, viruses, and the like present outside the body-might be reduced.

An object of the present invention is to provide a transdermal patch and a microneedle sheet that can efficiently introduce a target substance to the stratum corneum and prevent a reduction in the protection capability of the stratum corneum.

### TECHNICAL SOLUTION

Aspects of the present invention are explained below as the technical solution. These aspects can be arbitrarily combined as needed.

A microneedle sheet according to one aspect of the present invention contains a target substance to be introduced to the stratum corneum of the skin. The microneedle sheet comprises a sheet-shaped base material and a plurality of microneedles on the sheet-shaped base material. The plurality of microneedles are configured to make contact with the stratum corneum of the skin. Each microneedle of the plurality of microneedles includes a skin-stretching part projecting from the sheet-shaped base material. The skin-stretching part is configured to stretch the stratum corneum by pressing against the surface of the stratum corneum of the skin without sticking into the surface of the stratum corneum. The skin-stretching parts each have a tip surface, the surface area of which is 1 × 10⁻³ mm² or more and the height from the sheet-shaped base material to a front surface of which is 30 µm or more and 300 µm or less, and the skin-stretching parts are disposed spaced apart such that a spacing between boundaries of mutually adjacent skin-stretching parts and the sheet-shaped base material is greater than or equal to a value calculated by dividing the square of the height of the mutually adjacent skin-stretching parts by 100.

Based on the microneedle sheet configured in this manner, the skin-stretching part is configured to stretch the stratum corneum by pressing against the surface of the stratum corneum of the skin without sticking into the surface of the stratum corneum, thus, it is possible to prevent the skin-stretching part from breaking through the stratum corneum, and thereby to suppress a decrease in the protective ability of the stratum corneum. On the other hand, the sheet-shaped base material makes close contact with the stratum corneum of the skin, thus, it is possible to introduce the target substance with good efficiency from the sheet-shaped base material and the skin-stretching part to the inside of the stratum corneum stretched by the skin-stretching part.

A microneedle sheet according to another aspect of the present invention contains a target substance to be introduced to the stratum corneum of the skin. The microneedle sheet comprises a sheet-shaped base material and a plurality of microneedles on the sheet-shaped base material. The plurality of microneedles are configured to make contact with the stratum corneum of the skin. Each microneedle of the plurality of microneedles includes a skin-stretching part and a needlelike-projection part. A skin-stretching part projects from the sheet-shaped base material such that the skin-stretching part has a tip surface for stretching the stratum corneum by pressing against the surface of the stratum corneum of the skin without sticking into the surface of the stratum corneum. A needlelike-projection part on the tip surface of the skin-stretching part having a shape such that the needlelike-projection part sticks into the surface of the stratum corneum and a peak part of the needlelike-projection part stays inside the stratum corneum.

Based on the microneedle sheet configured in this manner, the skin-stretching part configured to stretch the stratum corneum by pressing against the surface of the stratum corneum of the skin without sticking into the surface of the stratum corneum, thus, a root of the needlelike-projection part at the skin-stretching part stays on the surface of the stratum corneum. Thereby, the needlelike-projection part can stick into the stratum corneum such that the peak part stays inside the stratum corneum and it is prevented to breaking through the stratum corneum by the needlelike-projection part. It is possible to introduce the target substance with good efficiency from the sheet-shaped base material and the skin-stretching part to the inside of the stratum corneum stretched by the skin-stretching part. On the other hand, it is possible to suppress a decrease in the protective ability of the stratum corneum. Furthermore, it is possible to introduce the target substance with good efficiency from the needlelike-projection part sticking into the surface of the stratum corneum to the inside of the stratum corneum stretched by the skin-stretching part.

It is acceptable that the needlelike-projection part has a surface area of less than 5 × 10⁻⁴ mm² in a plan view and a height of 1 µm or more and 20 µm or less. When the microneedle sheet is being affixed to the skin by an adhesive, the needlelike-projection part having such shape makes it easy for needlelike-projection part to stick into the stratum corneum and makes it easy for the peak part to stay inside the stratum corneum.

It is acceptable that the plurality of microneedles are disposed with a density wherein the surface area occupied by the needlelike-projection parts per unit of area is 0.2% or less. When the microneedle sheet is being affixed to the skin, by having such disposition of the plurality of microneedles, a sufficient pressure is applied to each of the needlelike-projection parts of the microneedles, thus, it is possible to improve reliability that the needlelike-projection parts penetrate into the inside of the stratum corneum.

It is acceptable that the plurality of microneedles is disposed with a density wherein the surface area occupied by the tip surfaces per unit of area is 0.3% or more. When the microneedle sheet is being affixed to the skin, by having such disposition of the plurality of microneedles, since an excessive pressure is not applied to each of the tip surfaces of the microneedles, the tip surfaces of the skin-stretching parts perform a function of suppressing the penetration into the inside of the stratum corneum.

It is acceptable that in the skin-stretching part, a length from the root of the needlelike-projection part to an end part of the tip surface is 8 µm or more and 20 µm or less. According to the skin-stretching part configured in this manner, a part from the root of needlelike-projection part to peripheral edge of the tip surface is a catch enough, thus, the tip surface is made hard to penetrate into the inside of the stratum corneum.

A transdermal patch according to one aspect of the present invention comprises the above-mentioned microneedle sheet and a pressing part that maintains a state in which the microneedle sheet is pressed against the skin. The transdermal patch comprising the pressing part can achieve a state that the skin-stretching part is stretching the stratum corneum by pressing against the surface of the stratum corneum of the skin without sticking into the surface of the stratum corneum. Thereby, it is easy to maintain a condition that the target substance is introduced with good efficiency from the sheet-shaped base material and the skin-stretching part to the inside of the stratum corneum stretched by the skin-stretching part for a long time.

### ADVANTAGEOUS EFFECTS

By using the transdermal patch and the microneedle sheet of the present invention, a tip surface of a skin-stretching part can push against the stratum corneum of the skin and thereby stretch the skin, and thereby a target substance can be introduced with good efficiency from a microneedle to the stratum corneum. When the skin is being stretched, the tip surface of the skin-stretching part tends not to tear the stratum corneum of the skin, which makes it possible to prevent a decrease in the protective capability of the stratum corneum.

### BRIEF DESCRIPTION OF DRAWINGS

FIG. 1 is a schematic cross-sectional view that shows a structural overview of a transdermal patch according to a first embodiment.
FIG. 2 is a schematic-enlarged side view for explaining a microneedle shown in FIG. 1.
FIG. 3 is a schematic-partial-enlarged side view of the microneedle in FIG. 2.
FIG. 4 is a schematic cross-sectional view for explaining the relationship between the microneedle and the skin according to the first embodiment.
FIG. 5 is a partial-broken-enlarged oblique view of a microneedle sheet for explaining the layout of the microneedles.
FIG. 6 includes views wherein (a) is a schematic cross-sectional view for explaining the relationship between the skin and a needlelike-projection part for the case in which a needlelike-projection part alone is provided on the sheet-shaped base material, (b) is a schematic cross-sectional view for explaining the relationship between the skin and the needlelike-projection part for the case in which a skin-stretching part is too large, and (c) is a schematic-partial-enlarged oblique view for explaining the relationship between the microneedle and the skin of the portion shown in FIG. 6(b).
FIG. 7 includes views wherein (a) is a schematic cross-sectional view for explaining the relationship between the skin and the needlelike-projection parts for the case in which the spacing between adjacent microneedles is too narrow, and (b) is a schematic cross-sectional view for explaining the relationship between the skin and the needlelike-projection parts for the case in which the spacing between adjacent microneedles is suitable.
FIG. 8 is a table for explaining the relationship between the spacing and the height of the microneedles.
FIG. 9 is a graph for explaining the relationship between the spacing and the height of the microneedles.
FIG. 10 is a schematic-partial-enlarged cross-sectional view for explaining the microneedle sheet according to a modified example of the first embodiment.
FIG. 11 is a schematic-enlarged side view for explaining the microneedle sheet according to a second embodiment.
FIG. 12 is a schematic-enlarged oblique view of the microneedle sheet in FIG. 11.
FIG. 13 is a schematic cross-sectional view for explaining the relationship between the microneedle and the skin according to the second embodiment.

### DESCRIPTION OF EMBODIMENTS

### <First Embodiment>

### (1) Overview of Structure of Transdermal Patch

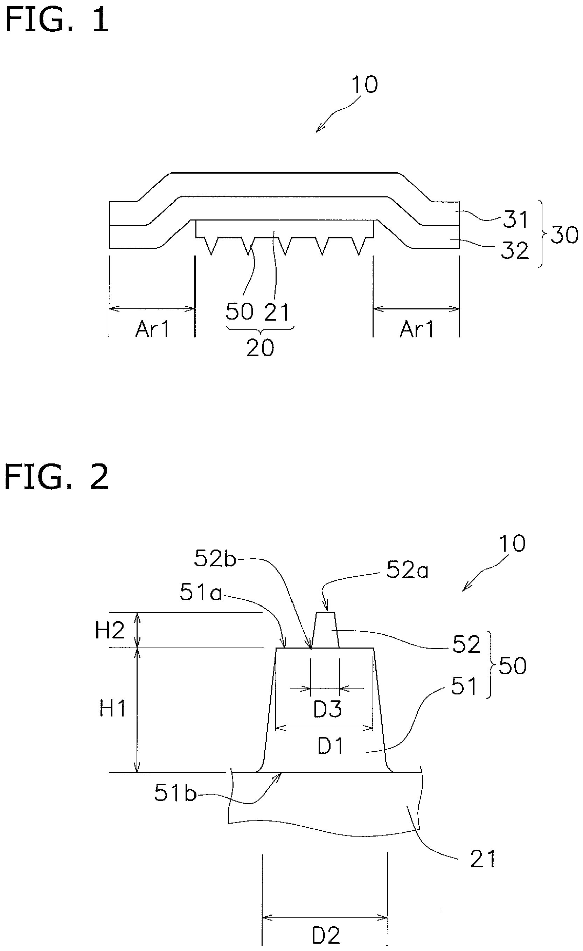

An overview of the structure of a transdermal patch according to a first embodiment of the present invention will now be explained, with reference to FIG. 1. FIG. 1 schematically shows a cross-sectional structure of the transdermal patch. A transdermal patch 10 shown in FIG. 1 comprises a microneedle sheet 20 and a moisture-permeable sheet 30. In the transdermal patch 10 in FIG. 1, an adhesive agent is applied to the entire surface of the moisture-permeable sheet 30, and thereby an adhesive layer 32 is formed. The moisture-permeable sheet 30 serves the function of a support member for maintaining the state in which the microneedle sheet 20 is affixed to the skin. The moisture-permeable sheet 30 is formed larger than the microneedle sheet 20 so that the moisture-permeable sheet 30 can sufficiently exhibit its function as a support member. If the microneedle sheet 20 is stuck on substantially the center of the moisture-permeable sheet 30, then the state results in which the moisture-permeable sheet 30 projects from the end part of the microneedle sheet 20 over the entire perimeter of the microneedle sheet 20. The adhesive agent, which is applied to an area Ar1 that projects from the moisture-permeable sheet 30, is affixed to the skin, and thereby the transdermal patch 10 is affixed to the skin in the state in which the microneedle sheet 20 is brought into contact with the skin.

### (1-1) Microneedle Sheet

The microneedle sheet 20 comprises a sheet-shaped base material 21 and a plurality of microneedles 50. In the microneedle sheet 20 shown in FIG. 1, a rear surface of the sheet-shaped base material 21 is affixed to the adhesive layer 32, and the plurality of microneedles 50 is formed on a front surface of the sheet-shaped base material 21.

The sheet-shaped base material 21 is molded into various planar shapes suited to the region of the skin at which the sheet-shaped base material 21 is to be stuck. The thickness of the sheet-shaped base material 21 is comparatively small, for example, several hundred microns. The sheet-shaped base material 21 may have a monolayer structure composed of a single material or may have a multilayer structure formed of differing materials.

The material of the front-surface layer of the sheet-shaped base material 21 is preferably the same as the material of the microneedles 50, as long as the state can be maintained in which the plurality of microneedles 50 stands upright on the front surface of the sheet-shaped base material 21. Specifically, the front-surface layer of the sheet-shaped base material 21 is formed of a biologically innocuous macromolecular substance. The biologically innocuous macromolecular substance contains, for example, a biologically innocuous resin, biologically innocuous polysaccharides, and biologically innocuous proteins, as well as biologically innocuous compounds derived therefrom. Here, biologically innocuous means biocompatible for medical care, cosmetic, or veterinary medical purposes when the amount introduced via the skin by a suitable usage method is appropriately adjusted.

The microneedles 50 are preferably formed of the same material as the material of the front-surface layer of the sheet-shaped base material 21, but a material suited to introducing the target substance to the stratum corneum is selected as the material of the microneedles 50. The material of the microneedles 50 is likewise formed of a biologically innocuous macromolecular substance. The macromolecular substance that serves as the material of the microneedles 50 likewise contains, for example, a biologically innocuous resin, biologically innocuous polysaccharides, and biologically innocuous proteins, as well as biologically innocuous compounds derived therefrom.

The biologically innocuous macromolecular substance that serves as the material of the microneedles 50 preferably contains at least one property among biosolubility and biodegradability. Here, biosolubility is a property in which a substance dissolves in vivo, and biodegradability is a property in which a substance decomposes in vivo. By virtue of the microneedles 50 being formed of a macromolecular substance having at least one of those two properties, the microneedles 50 introduced into the stratum corneum of the skin are subject to at least one action among dissolving and degrading in vivo and thus change gradually with the passage of time and consequently do not remain as a solid body in the stratum corneum for a long time.

The biosoluble and/or biodegradable macromolecular substance with which the microneedles 50 are formed is preferably water soluble. If the microneedles 50 introduced into the stratum corneum of the skin are a water-soluble macromolecular substance, then the microneedles 50 dissolve in the moisture inside the stratum corneum; therefore, it becomes easy to smoothly introduce the target substance into the stratum corneum using the microneedles 50.

For example, maltose, dextran, water-soluble chitosan, pullulan, sodium chondroitin sulfate, sodium hyaluronate, and glycogen can be given as examples of biologically innocuous polysaccharides and biologically innocuous compounds derived therefrom having water solubility and at least one property among biosolubility and biodegradability. For example, serum albumin and serum α-acid glycoprotein can be given as examples of biologically innocuous proteins and biologically innocuous compounds derived therefrom having water solubility and at least one property among biosolubility and biodegradability. For example, water soluble biologically innocuous biodegradable polymers and compounds derived therefrom can be given as examples of biologically innocuous resins and biologically innocuous compounds derived therefrom, having water solubility and at least one property among biosolubility and biodegradability. For example, carboxyvinyl polymer and block polymers having water solubility and biodegradability and in which polyethylene glycol (PEG) has been block copolymerized with poly(lactic-co-glycolic acid) (PLGA), polycaprolactone (PCL), or polylactic acid (PLA), can be given as examples of water soluble biologically innocuous biodegradable polymers and compounds derived therefrom. The PEG is water soluble biocompatible polymer.

In addition, for example, polylactic acid, polyglycolic acid, and polydioxane can be given as examples of biologically innocuous resins and biologically innocuous compounds derived therefrom having water insolubility and at least one property among biosolubility and biodegradability.

The materials used in the microneedles 50 described above can also be used, as is, as the material of the sheet-shaped base material 21.

When the material of the microneedles 50 is used as discussed above, the target substance introduced into the stratum corneum may be the same as the material described above but can also be a different material from the material described above. For example, a bioactive substance that is used for medical treatment, diagnosis, prevention, or the like of injury or illness or for cosmetic or veterinary medical purposes can be given as an example of a target substance introduced into the stratum corneum. Such a bioactive substance contains, for example, a drug, a nutrient, and a cosmetic. Because the microneedles 50 have a shape as described below, a bioactive substance that acts on the appearance of the stratum corneum or an injury or illness of the stratum corneum is particularly preferable as the target substance; for example, sodium hyaluronate, which has an effect on the cosmetic of the stratum corneum, can be given as an example.

For example, the entirety of the microneedle sheet 20 according to the first embodiment consists of sodium hyaluronate; for example, the entirety of the microneedles 50 likewise consists of sodium hyaluronate; and the transdermal patch 10 according to the first embodiment introduces the sodium hyaluronate to the stratum corneum of facial skin by, for example, spreading it on the face as a cosmetic.

Furthermore, the shape of the microneedles 50 will be explained in detail later.

### (1-2) Moisture-Permeable Sheet

The base material of the moisture-permeable sheet 30 is formed of, for example, a polyurethane film 31 having numerous (a plurality of) vapor-permeating holes (not shown), wherein the diameter of holes through which water vapor passes is from 0.1 µm to 100 µm and preferably is from 10 µm to 30 µm. The thickness of the moisture-permeable sheet 30 is, for example, approximately several tens of microns. In addition, the moisture-permeable sheet 30 has the adhesive layer 32 for sticking the moisture-permeable sheet 30 to the skin. The moisture-permeable sheet 30 is configured such that water vapor passes through the vapor-permeating holes of the polyurethane film 31 and the adhesive layer 32, and the skin at the location at which the moisture-permeable sheet 30 is affixed does not sweat. Consequently, for example, the adhesive layer 32 is applied sparsely so that the surface area of application is small and thus the vapor-permeating holes are not all plugged up by the adhesive layer 32.

### (2) Shape of Each Microneedle

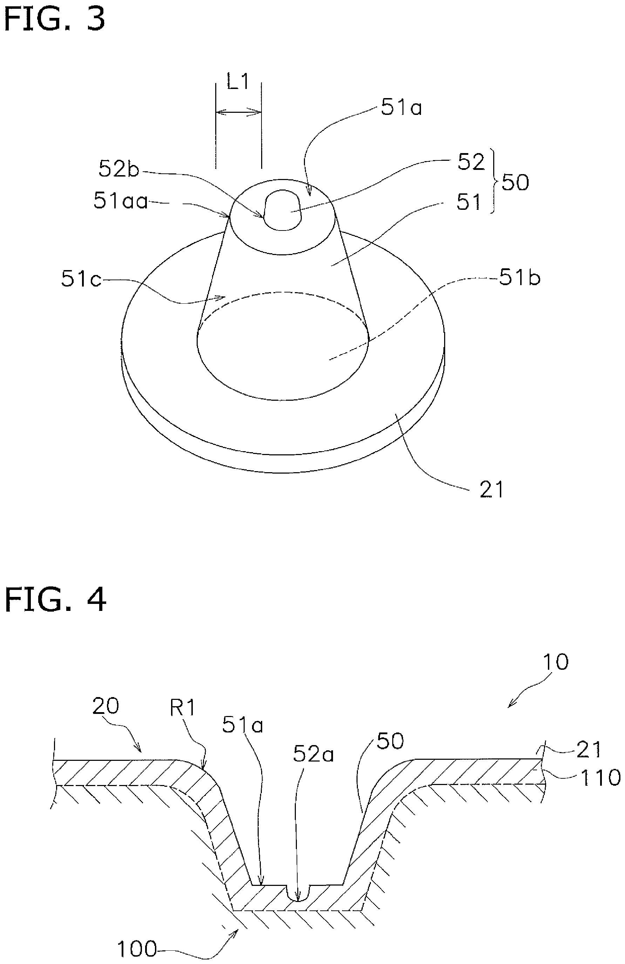

FIG. 2 is a partial-enlarged side view in which one microneedle and the vicinity thereof are partially enlarged. FIG. 3 is a partial-enlarged oblique view in which one microneedle and a portion of the vicinity thereof are enlarged. As shown in FIG. 2 and FIG. 3, the microneedle 50 has a skin-stretching part 51 and a needlelike-projection part 52. The skin-stretching part 51 is a portion that projects from the sheet-shaped base material 21 and is formed integrally with the sheet-shaped base material 21. The skin-stretching part 51 and the sheet-shaped base material 21 are, for example, formed entirely of sodium hyaluronate.

The skin-stretching part 51 is a circular truncated cone, wherein a diameter D1 of a tip surface 51a is set to within a range of 40-250 µm, a diameter D2 of a rear-part section 51b is set to within a range of 120-700 µm, and a height H1 from the rear-part section 51b to the tip surface 51a is set to within a range of 30-300 µm. The diameter D1 of the tip surface 51a is preferably set to within a range of 60-80 µm so that it is easy for the skin to deform. Expressing this in terms of the surface area of the tip surface 51 a, it is preferable that the surface area of the tip surface 51a, including the region in which the needlelike-projection part 52 is formed, is 1 × 10⁻³ mm² or more in order to reduce the pressure applied to the tip surface 51a and make it difficult for the tip surface 51a to penetrate into the inside of the stratum corneum.

The needlelike-projection part 52 is formed on the tip surface 51a of the skin-stretching part 51. The needlelike-projection part 52 has a conical shape, which narrows at its tip, a diameter D3 of the root part 52b is set to within a range of 1-24 µm, and a height H2 from the root part 52b to a peak part 52a is set to within a range of 1-20 µm. To make it such that the peak part 52a easily stays inside the stratum corneum and remains within the stratum corneum, it is preferable that the diameter D3 of the root part 52b is set to within a range of 5-18 µm and the height H2 from the root part 52b to the peak part 52a is set to within a range of 5-18 µm.

The shape of a horizontal section of the root part 52b of the needlelike-projection part 52 (a section parallel to the front surface of the sheet-shaped base material 21) is set to a size such that at least a length L1 from the root part 52b to an end part 51aa of the tip surface 51a of the skin-stretching part 51 fits within a range of 8-20 µm. Furthermore, there may be a plurality of the needlelike-projection parts 52 formed on one tip surface 51a; however, to reliably penetrate into the inside of the stratum corneum, there is preferably one needlelike-projection part 52. If expressed by the surface area of the root part 52b of the needlelike-projection part 52, that is, by the surface area of the needlelike-projection part 52 in a plan view, then, to increase the pressure applied to the needlelike-projection part 52 and to make it easy for the peak part 52a to penetrate into the inside of the stratum corneum, the surface area in a plan view is preferably less than 5 × 10⁻⁴ mm².

The skin-stretching part 51 set to the size as described above is configured such that, if the transdermal patch 10 is affixed to the skin of an average person by the adhesive layer 32, then the tip surface 51a of the skin-stretching part 51 does not penetrate into the stratum corneum of the skin. In other words, in the case of the shape of the skin-stretching part 51 set to the size as described above, when the transdermal patch 10 is affixed to the skin, the skin surrounding the skin-stretching part 51 stretches and the skin surface reaches the front surface of the sheet-shaped base material 21.

The needlelike-projection part 52 set to the size as described above is configured such that, if the transdermal patch 10 is affixed to the skin of an average person by the adhesive layer 32, then the peak part 52a of the needlelike-projection part 52 does not penetrate through the stratum corneum even if the entire needlelike-projection part 52 penetrates into the stratum corneum of the skin. In other words, in the case of the shape of the needlelike-projection part 52 set to the size as described above, in the state in which the skin surface reaches the front surface of the sheet-shaped base material 21, at least the peak part 52a of the needlelike-projection part 52 stays inside the stratum corneum and remains within the stratum corneum.

FIG. 4 schematically shows the relationship between one of the microneedles 50 and a skin 100 of a human when the transdermal patch 10 has been affixed to the skin 100. As can be understood from the cross section shown in FIG. 4, the skin-stretching part 51 does not penetrate into the inside of a stratum corneum 110, and only the needlelike-projection part 52 penetrates the interior of the stratum corneum 110. Furthermore, the peak part 52a of the needlelike-projection part 52 is located inside the stratum corneum 110.

### (3) Layout of Microneedles

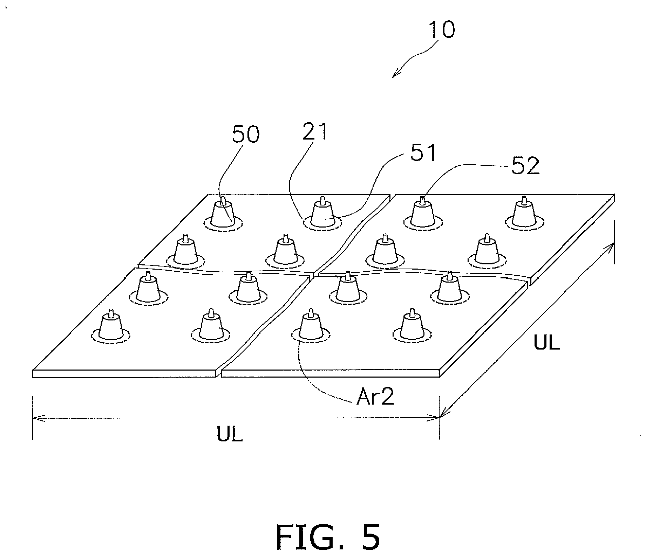

The plurality of microneedles 50 is, for example, arrayed in a grid as shown in FIG. 5. The plurality of microneedles 50 is not limited to being arrayed in a grid, but the microneedles 50 are overall preferably disposed evenly. This is because if there is bias in the layout of the microneedles 50, then a portion in which the pressure applied is comparatively high and a portion in which the pressure applied is comparatively low will adversely arise. This is because if the pressure differential between the comparatively high pressure portion and the comparatively low pressure portion becomes excessively large, then, for example, one region will arise in which the needlelike-projection parts 52 do not stick into the stratum corneum, the other region will arise in which, conversely, the skin-stretching parts 51 adversely stick into the stratum corneum, and the like.

When the transdermal patch 10 is being affixed, first, the needlelike-projection parts 52 contact the stratum corneum 110 of the skin 100; therefore, to make it easy for the plurality of needlelike-projection parts 52 to penetrate into the inside of the stratum corneum 110, it is preferable that high pressure is applied to every needlelike-projection part 52. Specifically, the root parts 52b are preferably disposed such that the aggregate of the surface areas of the root parts 52b disposed per unit of area becomes 0.2% or less (0.002 × UL² or less in FIG. 5). For example, if each root part 52b is circular and substantially flat and the diameter D3 is 24 µm, then 400/cm² or less should be disposed. Calculating the approximate aggregate surface area of the root parts 52b in this case yields 3.14 × 12 µm × 12 µm × 400 □ 0.18 mm² and 0.18 mm²/100 mm² × 100 = 0.18%, which satisfies the condition of 0.2% or less. If each root part 52b is rectangular, then, for example, 500/cm² or less of the needlelike-projection parts 52, wherein one side of each root part 52b is 20 µm, should be disposed.

In addition, to ensure that the plurality of the skin-stretching parts 51 does not penetrate into the inside of the stratum corneum, when the transdermal patch 10 is being stuck on, first, the needlelike-projection parts 52 contact the stratum corneum 110 of the skin 100, after which the tip surfaces 51 a of the skin-stretching parts 51 contact the stratum corneum 110 of the skin 100, and therefore it is preferable to distribute, to as many of the skin-stretching parts 51 as possible, the pressure that is applied when the skin deforms. Specifically, the tip surfaces 51 a are preferably arranged such that the aggregate of the surface areas of the tip surfaces 51a per unit of area becomes 0.3% or more of the unit of area (e.g., 0.003 × UL² or more in FIG. 5). For example, if the tip surface 51a is circular and substantially flat and the diameter D1 is 40 µm, then 250/cm² or more should be disposed. Calculating the approximate aggregate surface area of the tip surfaces 51a in this case yields 3.14 × 20 µm × 20 µm × 250 □ 0.31 mm² and 0.31 mm²/100 mm² × 100 = 0.31%, which satisfies the condition of 0.3% or more of the unit of area.

In addition, as explained previously, to make the pressure that is applied when the skin deforms small, the diameter D1 of the tip surface 51a is set to within a range of 40-250 µm.

Furthermore, to sufficiently stretch the skin, the height H1 from the rear-part section 51b to the tip surface 51a is set to within a range of 30-300 µm.

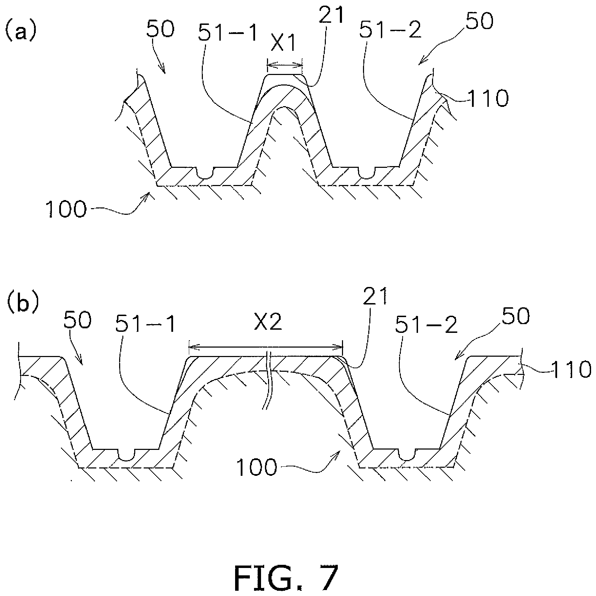

For example, when the transdermal patch 10 is affixed to the skin 100, areas Ar2, each of which is surrounded by a chain line circle and the rear-part section 51b of the microneedle 50 as shown in FIG. 5, are areas that do not contact the skin 100. To prevent the stratum corneum 110 from being injured by the skin-stretching parts 51 even if high pressure is applied to the transdermal patch 10, in such non-contacting areas Ar2, the aggregate surface area is preferably 0.3% or less of the unit of area (e.g., 0.003 × UL² or less in FIG. 5) when a pressure of 0.5 MPa is applied to the microneedle sheet 20. To that end, they are preferably disposed such that a spacing between the boundaries of mutually adjacent skin-stretching parts and the sheet-shaped base material is greater than or equal to the value calculated by dividing the square of the height of the mutually adjacent skin-stretching parts by 100. This point is explained later in detail, with reference to FIG. 7 to FIG. 9. In addition, it is preferable to make the tilt of a side surface 51c of the skin-stretching part 51 gentle, and it is preferable that the difference between the diameter D2 of the rear-part section 51b and the diameter D1 of the tip surface 51a is 100 µm or more.

Furthermore, the microneedle 50 is formed into a shape in which the surface of the skin 100 makes substantially close contact with the microneedle 50 and the front surface of the sheet-shaped base material 21 when a pressure of approximately 0.13 MPa is applied. To make the close contact with the skin 100 better at this time, a radius of curvature R1 of the base portion of the rear-part section 51b is preferably 0.1 mm or less and 0.01 mm or more and more preferably 0.05 mm or less.

Because the portion at which the skin 100 makes contact with the sheet-shaped base material 21 in this manner becomes large, it is preferable, considering also transdermal administration from the sheet-shaped base material 21 to the skin 100, that the microneedle 50 and the sheet-shaped base material 21 are composed of the same material.

### (4) Microneedle-Sheet-Manufacturing Method

The microneedle sheet 20 is manufactured by pouring an aqueous solution of sodium hyaluronate into a stamper (not shown), which is a mold wherein the shapes of the plurality of microneedles 50 have been engraved, and then drying such. If sodium hyaluronate is the target substance to be administered, then the microneedle sheet 20 may be formed using sodium hyaluronate as the principal component. In addition, if the target substance is, for example, sodium hyaluronate and another bioactive substance, then the microneedle sheet 20 may be manufactured by pouring, into the stamper, an aqueous solution of sodium hyaluronate to which the other bioactive substance has been added, and then drying such. Furthermore, substances other than the target substance, such as a target substance stabilizer, may also be added. In addition, methods other than those mentioned above may be used as the method of adding other bioactive substances to the sodium hyaluronate; for example, other bioactive substances can also be added by applying them after the drying.

Based on the manufacturing method described above, the microneedle sheet 20 can be manufactured wherein, for example, 250/cm of the microneedles 50 consisting of sodium hyaluronate are formed. This microneedle sheet 20 comprises: the skin-stretching parts 51, wherein the diameter D1 of the tip surface 51a is 68 µm, the diameter D2 of the rear-part section 51b is 200 µm, and the height H1 is 100 µm; and the needlelike-projection parts 52, wherein the diameter D3 of the root part 52b is 20 µm, the height H2 is 15 µm, and the length L1 is 8 µm.

### (5) Features

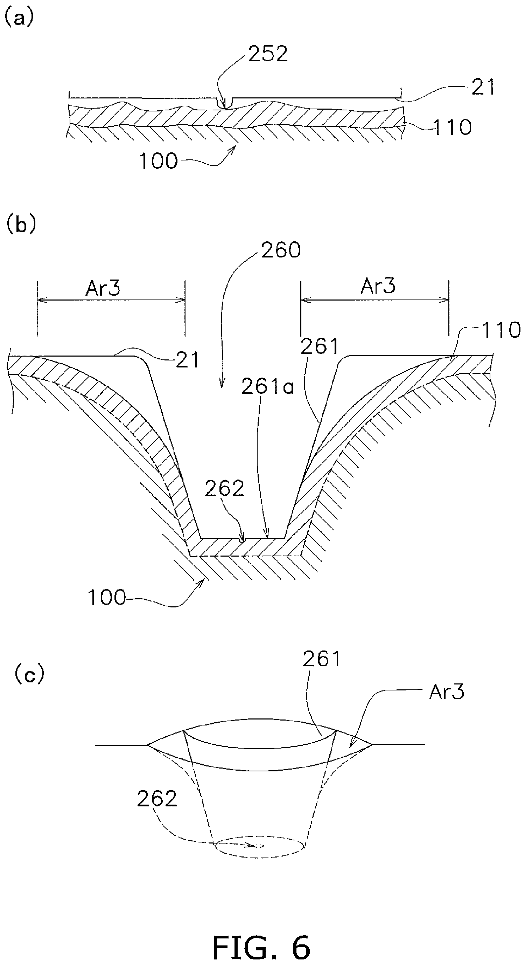

FIG. 6(a) is a schematic cross-sectional view that shows the state immediately before one, wherein a needlelike-projection part 252 has been formed on the sheet-shaped base material 21, is brought into contact with the skin 100. As shown in FIG. 6(a), if the needlelike-projection part 252 is formed directly on the flat sheet-shaped base material 21, a situation adversely arises in which, with just an extremely fine needlelike-projection part 252, the stratum corneum 110 is not pierced well because the skin 100 stretches due to the unevenness, waviness, structure, or the like of the skin 100, or a situation adversely arises in which the needlelike-projection part 252 does not contact the surface of the skin 100, owing to the unevenness, waviness, or the like of the skin 100.

In contrast, if the skin-stretching part 51 shown in FIG. 4 is present, then the state results in which the skin 100 is stretched out by the skin-stretching part 51 and the stretch percentage of the needlelike-projection part 52 decreases compared with the state before the transdermal patch 10 is affixed; consequently, even in the fine needlelike-projection part 52 in which the height is from 1 µm to 20 µm, the peak part 52a thereof penetrates into the inside of as far as the interior of the stratum corneum 110.

As a result, the sodium hyaluronate is supplied to the stratum corneum 110 not only from the front surface of the sheet-shaped base material 21 and the tip surface 51a and side surface 51c of the skin-stretching part 51 but also from the needlelike-projection part 52, and therefore the amount of the sodium hyaluronate supplied to the stratum corneum 110 can be increased more than the case of the skin-stretching part 51 alone without the needlelike-projection part 52.

Moreover, when the transdermal patch 10 is to be used, the circumstance frequently arises in which, for example, even when retiring to bed, the transdermal patch 10 is left stuck on the skin by the adhesive layer 32 (an example of a pressing part). It is expected that, when retiring to bed, a user using the transdermal patch 10 will unconsciously strike the transdermal patch 10 against the bedding, the surrounding fixtures, and the like, thereby applying a high pressure to the transdermal patch 10. If, as shown in FIG. 6(b) and FIG. 6(c), a skin-stretching part 261 is too tall, then a circumstance will arise in which an area Ar3, wherein the skin-stretching part 261, the front surface of the sheet-shaped base material 21, and the like do not contact the skin 100, will become too large, and a high pressure will adversely be applied to a tip surface 261 a of a microneedle 260; consequently, there will be a greater possibility that a case will occur in which the stratum corneum 110 is torn by the tip surface 261 a. As shown in FIG. 7(b), the microneedles 50 of the first embodiment described above are disposed spaced apart such that a spacing X2 between the boundaries of mutually adjacent skin-stretching parts 51-1, 51-2 and the sheet-shaped base material 21 is greater than or equal to the value calculated by dividing the square of the height H1 of the mutually adjacent skin-stretching parts by 100, that is, spaced apart by X2 □ (HI × H1) + 100. The non-contacting areas Ar2, wherein the stratum corneum 110 does not contact the sheet-shaped base material 21, are configured such that the aggregate surface area is 0.3% or less of the unit of area (e.g., 0.003 × UL² or less in FIG. 5) when a pressure of 500 gf/cm² is applied to the microneedle sheet 20. In addition, to make the tilt of the side surface 51c of each skin-stretching part 51 gentle, the difference between the diameter D2 of the rear-part section 51b and the diameter D1 of the tip surface 51a is set to 132 µm (≥ 100 µm). If a spacing X1 (<< X2) between the boundaries of the skin-stretching parts 51-1, 51-2 and the sheet-shaped base material 21 is small, as shown in FIG. 7(a), then there is an increased possibility that the area in which the stratum corneum 110 does not contact the sheet-shaped base material 21 will increase and that the microneedles 50 will break through and pass through the stratum corneum 110 when a high pressure is applied to the microneedle sheet 20.

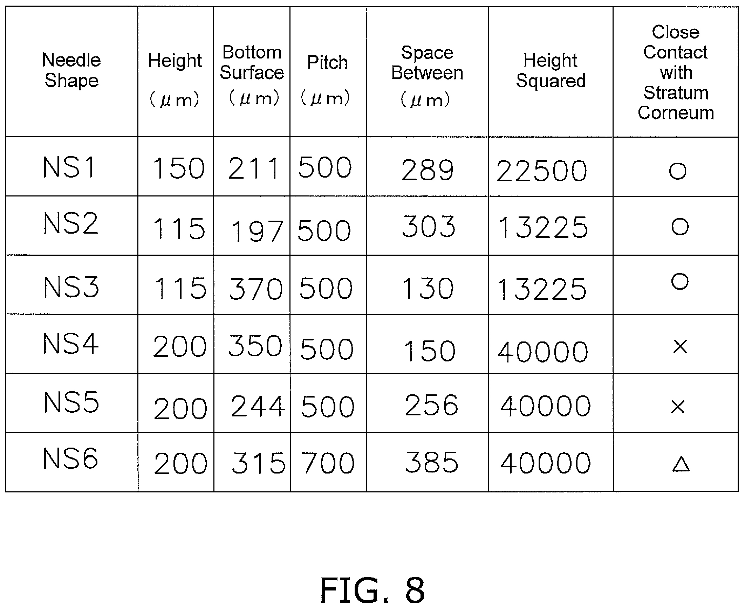

FIG. 8 lists examples that show the relationship, for the microneedle sheet 20 formed of sodium hyaluronate, between the shape of the microneedles 50 and the making of close contact with the stratum corneum 110 of the skin 100 by the microneedle sheet 20. In FIG. 8, "O" recorded in the "Close Contact with Skin" column indicates that the close contact with the skin is satisfactory, "X" indicates that the close contact with the skin is unacceptable, and "Δ" indicates that close contact with the skin is not very good. Furthermore, in needle shapes NS4, NS5, NS6 listed in FIG. 8, the skin-stretching parts 51 were seen to stick into the stratum corneum 110. If the skin-stretching parts 51 stick into the stratum corneum 110, then the distance between the stratum corneum 110 and the sheet-shaped base material 21 becomes close; however, even so, the close contact with the stratum corneum 110 was not satisfactory, and the needle shapes NS4, NS5, NS6 are listed as examples in which close contact was not good.

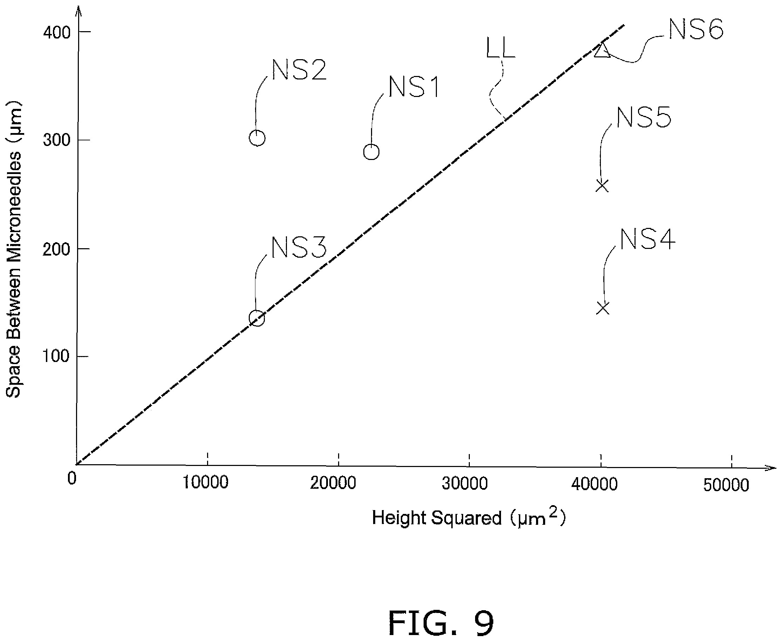

Incidentally, if the height of the skin-stretching part 51 is "0 µm," then the sheet-shaped base material 21 makes close contact with the stratum corneum 110, and therefore, as shown by the graph in FIG. 9, it is considered that a straight line LL, which indicates the range in which close contact is satisfactory, passes through the origin of the graph. Furthermore, if the height range of the skin-stretching parts 51 is within a narrow range such as 30-300 µm, then, even if the line LL approximates a straight line, it is not considered a problem. Accordingly, if a straight line is drawn that passes through the origin and passes between those in which close contact is satisfactory and those in which close contact is not very good, then a graph like that in FIG. 9 is obtained. Based on this straight line LL, it is understood that, if the condition is satisfied wherein (the space between adjacent microneedles 50) (≥ (the square of the height of the microneedles 50) ÷ 100, then the microneedle sheet 20 wherein close contact with the stratum corneum 110 is good will be obtained. Furthermore, "space between mutually adjacent microneedles 50" recited herein is the "spacing X2 between the boundaries of the mutually adjacent skin-stretching parts 51-1, 51-2 and the sheet-shaped base material 21" shown in FIG. 7(b).

Furthermore, because the pressure applied to one of the microneedles 50 becomes large if the space between the microneedles 50 widens, rather than increase the height of the microneedles 50 and widen the spaces therebetween, it is preferable to shorten the height of the microneedles 50 and narrow the spaces therebetween. Viewing FIG. 9 from such a viewpoint, it is considered preferable that the square of the height of the microneedles 50 be 40,000 or less. In addition, if the heights of mutually adjacent microneedles 50 differ and there are tall and short ones, then accord with the tall ones.

In addition, the extent of close contact between the stratum corneum 110 and the microneedle sheet 20, injury to the stratum corneum 110 caused by the skin-stretching parts 51, the penetration of the stratum corneum 110 by the needlelike-projection parts 52, and the like were evaluated by observing an enlarged image, produced by an optical observing apparatus, of the microneedle sheet 20 at the portion at which it is actually affixed to the skin.

Furthermore, because the radius of curvature R1 of the base portion of the rear-part section 51b is set to 5 µm (≥ 3 µm), the microneedles 50 are formed into a shape in which, when a pressure of approximately 0.13 MPa is applied, the surface of the skin 100 makes substantially close contact with the microneedles 50 and the front surface of the sheet-shaped base material 21, and thereby close contact with the skin 100 is improved.

As shown in FIG. 5, the microneedles 50 are formed into a shape in which the surface of the skin 100 makes substantially close contact with the microneedles 50 and the front surface of the sheet-shaped base material 21 when a force of 10 N is applied, in other words, when a pressure of approximately 0.13 MPa is applied, to a disk, having a diameter of 10 mm, on which the plurality of microneedles 50 are formed. Because the microneedles 50 have such a shape, even if a pressure higher than 0.13 MPa is applied to the microneedle sheet 20, most of the pressure impacts the sheet-shaped base material 21 and stress is not concentrated on the tip surfaces 51 a, which prevents tearing of the stratum corneum 110 by the tip surfaces 51a.

In addition, if the portion at which the skin 100 makes close contact with the sheet-shaped base material 21 becomes large, then it is preferable, considering also transdermal administration from the sheet-shaped base material 21 to the skin 100, that the microneedle 50 and the sheet-shaped base material 21 are composed of the same material. For example, when it is desired to administer as much sodium hyaluronate as possible to the skin 100 for cosmetic purposes, it is effective for the sheet-shaped base material 21 to be formed of sodium hyaluronate.

### (6) Modified Example

### (6-1) Modified Example 1A

The abovementioned first embodiment explained an exemplary case in which the microneedle sheet 20 is formed of sodium hyaluronate; however, the microneedle sheet 20 can also be formed of, for example, a thermoplastic that differs from that of the first embodiment. The microneedle sheet in such a case may be manufactured by injection molding in a mold in which shapes the same as those of the plurality of microneedles 50 have been engraved. If the target substance is not a thermoplastic, then, as long as the target substance does not degenerate with heat, the target substance can be mixed into the thermoplastic and the result can then be injection molded, or the target substance may be added to the thermoplastic after molding by a means such as applying it after injection molding. If the target substance is sensitive to heat or is expensive, then the cost incurred when adding the target substance can be kept down by, for example, applying the target substance after the injection molding.

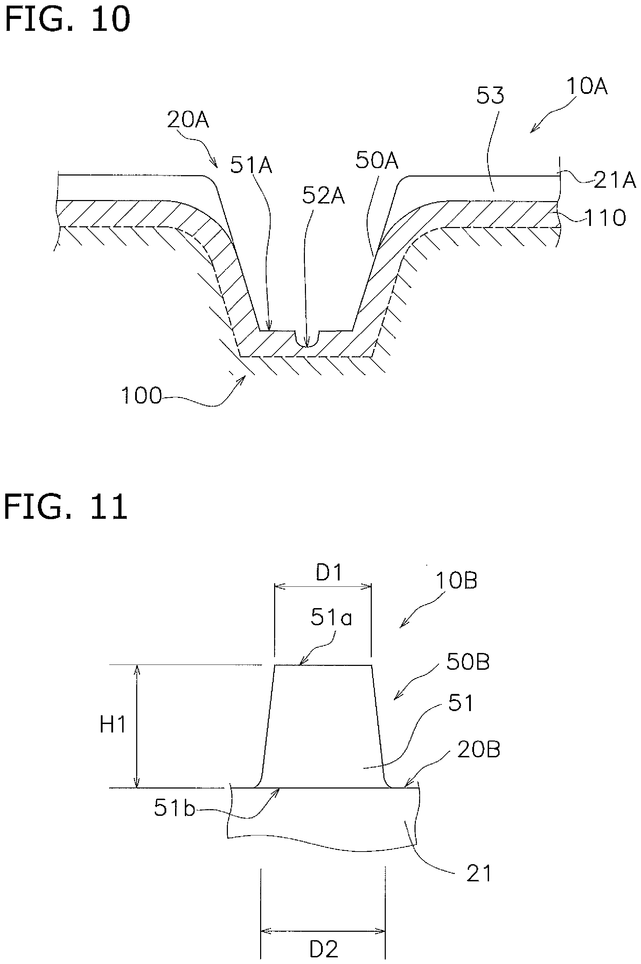

For example, as in a microneedle sheet 20A of a transdermal patch 10A shown in FIG. 10, a sheet-shaped base material 21A and a microneedle 50A are formed of, for example, polylactic acid, which is a thermoplastic, and a layer 53 of sodium hyaluronate may be applied thereupon. At this time, the skin-stretching part 51 and the needlelike-projection part 52 of the microneedle 50A are also formed of polylactic acid. In this case, because the microneedle 50A does not dissolve in water, like the microneedle 50 composed of sodium hyaluronate, the shape of the microneedle 50A can be maintained even when affixed to the skin 100.

### (6-2) Modified Example 1B

The abovementioned first embodiment explained an exemplary case in which, if the target substance is sodium hyaluronate, then the microneedle sheet 20 is formed of sodium hyaluronate; however, for example, if the target substance is a medicine, then the medicine may be added to the microneedle sheet 20 formed of sodium hyaluronate, or the microneedle sheet 20 may be coated with the medicine.

### (6-3) Modified Example 1C

The abovementioned embodiment explained an exemplary case in which the adhesive layer 32, which is formed on the moisture-permeable sheet 30 that covers the microneedle sheet 20, serves as the pressing part, which maintains the state in which the microneedle sheet 20 is pressed against the skin 100; however, the pressing part is not limited to the adhesive agent of such a mode. For example, an adhesive agent, a bonding agent, or the like formed on the front surface of the sheet-shaped base material 21 of the microneedle sheet 20, that is, formed on the surface on which the microneedles 50 are formed, can also serve as the pressing part. In addition, the pressing part may be a bandage, wherein the microneedle sheet 20 is wrapped to a human body in the state in which the microneedle sheet 20 makes close contact with the skin 100. In the case of a bandage, the bandage may be configured such that the pressing force is increased by the provision of a cushion member at the portion that contacts the microneedle sheet 20. In addition, the microneedle sheets 20 may be affixed to portions of a patch, as in an eye patch, wherein a string-shaped member, such as a string or an elastic band, is affixed to both ends of the patch, and the string-shaped member is fastened to the body or hooked onto the body.

### <Second Embodiment>

### (7) Overview of Structure of Transdermal Patch

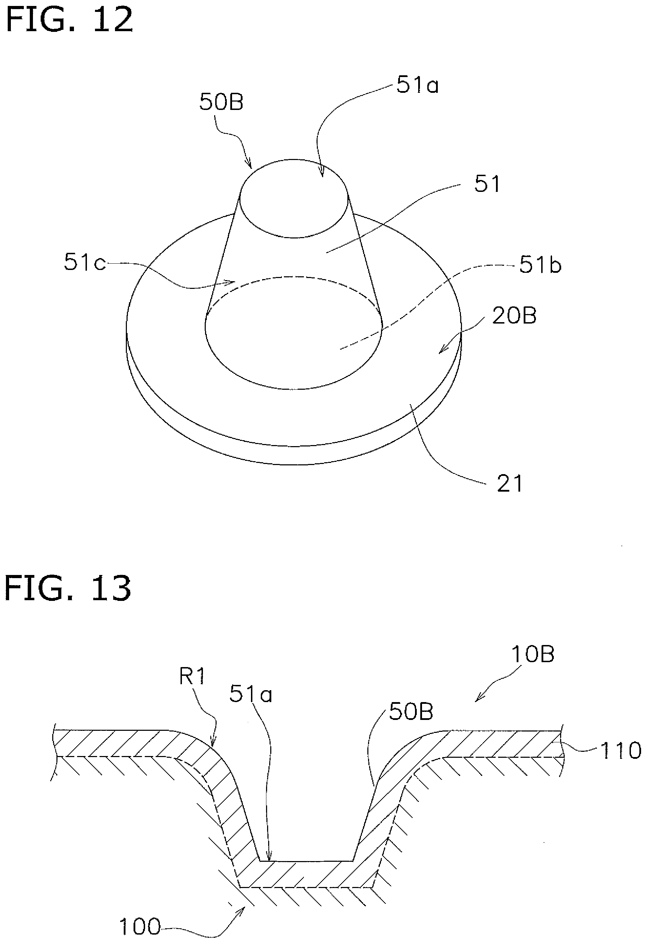

A transdermal patch 10B according to a second embodiment will now be explained, with reference to FIG. 11 to FIG. 13. FIG. 11 is a partial-enlarged side view in which one microneedle and a portion of the vicinity thereof are enlarged. FIG. 12 is a partial-enlarged oblique view in which the one microneedle and a portion of the vicinity thereof are enlarged. As shown in FIG. 11 and FIG. 12, in the transdermal patch 10B according to the second embodiment, a microneedle 50B comprises the skin-stretching part 51. However, unlike the transdermal patch 10 according to the abovementioned first embodiment, a needlelike-projection part is not formed on the tip surface 51a of the skin-stretching part 51. Other than the shape and layout of the plurality of microneedles 50B, the transdermal patch 10B according to the second embodiment is configured the same as the transdermal patch 10 according to the first embodiment and therefore the shape and layout of the microneedles 50B of the transdermal patch 10B according to the second embodiment are explained below, and other explanations are omitted.

### (8) Shape of Each Microneedle

As shown in FIG. 11 and FIG. 12, the microneedle 50B comprises the skin-stretching part 51, which projects from the sheet-shaped base material 21, and is molded integrally with the sheet-shaped base material 21. The skin-stretching part 51 and the sheet-shaped base material 21 are, for example, formed entirely of sodium hyaluronate.

The skin-stretching part 51 is a circular truncated cone, wherein the diameter D1 of a tip surface 51a is set to within a range of 40-250 µm, the diameter D2 of a rear-part section 51b is set to within a range of 120-700 µm, and the height H1 from the rear-part section 51b to the tip surface 51a is set to within a range of 30-300 µm. The diameter D1 of the tip surface 51a is preferably set to within a range of 60-80 µm so that it is easy for the skin to deform. Expressing this in terms of the surface area of the tip surface 51 a, it is preferable that the surface area of the tip surface 51a is 1 × 10⁻³ mm² or more in order to reduce the pressure applied to the tip surface 51a and make it difficult for the tip surface 51a to penetrate into the inside of the stratum corneum.

The skin-stretching part 51 set to the size as described above is configured such that, if the transdermal patch 10B is affixed to the skin of an average person, then the tip surface 51 a of the skin-stretching part 51 does not penetrate into the inside of the interior of the stratum corneum of the skin. In other words, in the case of the shape of the skin-stretching part 51 set to the size as described above, the skin surrounding the skin-stretching part 51 stretches and the skin surface reaches the front surface of the sheet-shaped base material 21 when the transdermal patch 10B is affixed to the skin.

FIG. 13 schematically shows the relationship between one of the microneedles 50B and the skin 100 of a human when the transdermal patch 10B has been affixed to the skin 100. As can be understood from the cross section shown in FIG. 13, the skin-stretching part 51 does not penetrate into the inside of the stratum corneum 110, and therefore only the stratum corneum 110 surrounding that is stretched.

### (9) Layout of Microneedles

The plurality of microneedles 50B is, for example, arrayed in a grid in the same way as the plurality of microneedles 50 shown in FIG. 5. The plurality of microneedles 50B is not limited to being arrayed in a grid, but the microneedles 50B are overall preferably disposed evenly. This is because if there is bias in the layout of the microneedles 50B, then a portion in which the pressure applied is comparatively high and a portion in which the pressure applied is comparatively low will adversely arise. This is because if the pressure differential between the comparatively high pressure portion and the comparatively low pressure portion becomes excessively large, then, for example, a region will arise in which, conversely, the skin-stretching parts 51 adversely stick into the stratum corneum, and the like.

To ensure that the plurality of the skin-stretching parts 51 does not penetrate into the inside of the stratum corneum, when the transdermal patch 10B is being affixed, first, the tip surfaces 51a of the skin-stretching parts 51 contact the stratum corneum 110 of the skin 100, and therefore it is preferable to distribute, to as many of the skin-stretching parts 51 as possible, the pressure that is applied when the skin deforms. Specifically, the tip surfaces 51a are preferably arranged such that the aggregate of the surface areas of the tip surfaces 51a per unit of area becomes 0.3% or more of the unit of area (e.g., 0.003 × UL² or more in FIG. 5).

The microneedles 50B of the second embodiment described above are disposed spaced apart such that a spacing between the boundaries of mutually adjacent skin-stretching parts 51 and the sheet-shaped base material 21 is greater than or equal to the value calculated by dividing the square of the height of the mutually adjacent skin-stretching parts by 100; in the example described above, disposed spaced apart by 200 µm or more (HI × 2). Based on such a configuration, the non-contacting areas Ar2, wherein the stratum corneum 110 does not contact the sheet-shaped base material 21, are configured such that the aggregate surface area is 0.3% or less of the unit of area (e.g., 0.003 × UL² or less in FIG. 5) when a pressure of 500 gf/cm² is applied to the microneedle sheet 20. As a result, it is possible to prevent the stratum corneum 110 from being injured by the skin-stretching part 511 even if high pressure is applied to the transdermal patch 10B. In addition, to make the tilt of the side surface 51c of each skin-stretching part 51 gentle, the difference between the diameter D2 of the rear-part section 51b and the diameter D1 of the tip surface 51a is set to 132 µm (≥ 100 µm). Making the tilt of the side surface 51c gentle decreases the possibility that the microneedles 50B will break through and pass through the stratum corneum 110 when high pressure is applied to a microneedle sheet 20B.

Furthermore, because the radius of curvature R1 of the base portion of the rear-part section 51b is set to 0.05 mm, the microneedles 50B are formed into a shape in which, when a pressure of approximately 0.13 MPa is applied, the surface of the skin 100 makes substantially close contact with the microneedles 50B and the front surface of the sheet-shaped base material 21, and thereby close contact with the skin 100 is improved.

The plurality of microneedles 50B of the second embodiment are formed into a shape in which the surface of the skin 100 makes substantially close contact with the microneedles 50B and the front surface of the sheet-shaped base material 21 when a pressure of approximately 0.13 MPa is applied. Because the microneedles 50B have such a shape, even if a pressure higher than 0.13 MPa is applied to the microneedle sheet 20B, most of the pressure impacts the sheet-shaped base material 21 and stress is not concentrated on the tip surfaces 51 a, which prevents tearing of the stratum corneum 110 by the tip surfaces 51a.

Because the portion at which the skin 100 makes contact with the sheet-shaped base material 21 in this manner becomes large, it is preferable, considering also transdermal administration from the sheet-shaped base material 21 to the skin 100, that the microneedle 50B and the sheet-shaped base material 21 are composed of the same material.

### (10) Features

In the microneedles 50B of the second embodiment described above, the microneedles 50B are formed into a shape in which the surface of the skin 100 makes substantially close contact with the microneedles 50B and the front surface of the sheet-shaped base material 21 when a force of 10 N is applied, in other words, when a pressure of approximately 0.13 MPa is applied, to a disk, having a diameter of 10 mm, on which the plurality of microneedles 50B are formed. Because the microneedles 50B have such a shape, even if a pressure higher than 0.13 MPa is applied to the microneedle sheet 20, most of the pressure impacts the sheet-shaped base material 21 and stress is not concentrated on the tip surfaces 51 a, which prevents tearing of the stratum corneum 110 by the tip surfaces 51a.

In addition, if the portion at which the skin 100 makes close contact with the sheet-shaped base material 21 becomes large, then it is preferable, considering also transdermal administration from the sheet-shaped base material 21 to the skin 100, that the microneedle 50B and the sheet-shaped base material 21 are composed of the same material. For example, when it is desired to administer as much sodium hyaluronate as possible to the skin 100 for cosmetic purposes, it is effective for the sheet-shaped base material 21 to be formed of sodium hyaluronate.

Comparing the case in which the front surface of the sheet-shaped base material 21 and the microneedle sheet 20B, whereon the skin-stretching part 51 is formed, is affixed to the skin with the case in which the sheet-shaped base material 21 alone is affixed to the skin as described above, because the sodium hyaluronate is supplied to the stratum corneum 110 from the tip surface 51 a and the side surface 51c of the skin-stretching part 51, the amount of sodium hyaluronate supplied to the stratum corneum 110 by the microneedle sheet 20B is greater than the one comprising only the sheet-shaped base material 21, wherein the sodium hyaluronate is supplied to the stratum corneum 110 from the sheet-shaped base material alone.

### (11) Modified Examples

### (11-1) Modified Example 2A

The abovementioned second embodiment explained an exemplary case in which the microneedle sheet 20B is formed of sodium hyaluronate; however, the microneedle sheet 20B can also be formed of, for example, a thermoplastic that differs from that of the first embodiment. The microneedle sheet in such a case may be manufactured by injection molding in a mold in which shapes the same as those of the plurality of microneedles 50B have been engraved. If the target substance is not a thermoplastic, then, as long as the target substance does not degenerate with heat, the target substance can be mixed into the thermoplastic and the result can then be injection molded, or the target substance may be added to the thermoplastic after molding by a means such as applying it after injection molding. If the target substance is sensitive to heat or is expensive, then the cost incurred when adding the target substance can be kept down by, for example, applying the target substance after the injection molding. For example, the transdermal patch 10A shown in FIG. 7 can also be configured such that the needlelike-projection parts 52 are removed.

### (11-2) Modified Example 2B

The abovementioned second embodiment explained an exemplary case in which, if the target substance is sodium hyaluronate, which is used to beautify human skin, then the microneedle sheet 20B is formed of sodium hyaluronate; however, for example, if the target substance is a medicine, then the medicine may be added to the microneedle sheet 20B formed of sodium hyaluronate, or the microneedle sheet 20B may be coated with the medicine.

### (11-3) Modified Example 2C

As in the above-mentioned first embodiment, the adhesive layer 32, which is formed on the moisture-permeable sheet 30 that covers the microneedle sheet 20B, serves as the pressing part, which maintains the state in which the microneedle sheet 20B is pressed against the skin 100; however, the pressing part is not limited to the adhesive agent of such a mode. For example, an adhesive agent, a bonding agent, or the like formed on the front surface of the sheet-shaped base material 21 of the microneedle sheet 20B, that is, formed on the surface on which the microneedles 50B are formed, can also serve as the pressing part. In addition, the pressing part may be a bandage, wherein the microneedle sheet 20B is wrapped to a human body in the state in which the microneedle sheet 20B makes close contact with the skin 100. In the case of a bandage, the bandage may be configured such that the pressing force is increased by the provision of a cushion member at the portion that contacts the microneedle sheet 20B. In addition, the microneedle sheets 20B may be affixed to portions of a patch, as in an eye patch, wherein a string-shaped member, such as a string or an elastic band, is affixed to both ends of the patch, and the string-shaped member is fastened to the body or hooked onto the body.

### REFERENCE SIGNS LIST

- 10, 10A, 10B: Transdermal patch
- 20, 20A, 20B: Microneedle sheet
- 21, 21A: Sheet-shaped base material
- 30: Moisture-permeable sheet
- 31: Polyurethane film
- 32: Adhesive layer
- 50, 50A, 50B: Microneedle
- 51: Skin-stretching part
- 52: Needlelike-projection part

## Claims

1. A microneedle sheet containing a target substance to be introduced to a stratum corneum of a skin, the microneedle sheet comprising:
a sheet-shaped base material; and
a plurality of microneedles on the sheet-shaped base material, the plurality of microneedles configured to make contact with the stratum corneum of the skin; wherein
each microneedle of the plurality of microneedles includes:
a skin-stretching part projecting from the sheet-shaped base material, the skin-stretching part configured to stretch the stratum corneum by pressing against the surface of the stratum corneum of the skin without sticking into the surface of the stratum corneum; wherein
the skin-stretching parts each have a tip surface, the surface area of which is 1 × 10⁻³ mm² or more and the height from the sheet-shaped base material to a front surface of which is 30 µm or more and 300 µm or less, and the skin-stretching parts are disposed spaced apart such that a spacing between boundaries of mutually adjacent skin-stretching parts and the sheet-shaped base material is greater than or equal to a value calculated by dividing the square of the height of the mutually adjacent skin-stretching parts by 100.

2. A microneedle sheet containing a target substance to be introduced to a stratum corneum of a skin, the microneedle sheet comprising:
a sheet-shaped base material; and
a plurality of microneedles on the sheet-shaped base material, the plurality of microneedles configured to make contact with the stratum corneum of the skin; wherein
each microneedle of the plurality of microneedles includes:
a skin-stretching part projecting from the sheet-shaped base material such that the skin-stretching part has a tip surface for stretching the stratum corneum by pressing against the surface of the stratum corneum of the skin without sticking into the surface of the stratum corneum; and
a needlelike-projection part on the tip surface of the skin-stretching part, the needlelike-projection part having a shape such that the needlelike-projection part sticks into the surface of the stratum corneum and a peak part of the needlelike-projection part stays inside the stratum corneum.

3. The microneedle sheet according to claim 2, wherein
the needlelike-projection part has a surface area of less than 5 × 10⁻⁴ mm² in a plan view and a height of 1 µm or more and 20 µm or less.

4. The microneedle sheet according to claim 3, wherein
the plurality of microneedles are disposed with a density wherein the surface area occupied by the needlelike-projection parts per unit of area is 0.2% or less.

5. The microneedle sheet according to any one of claim 2 through claim 4, wherein
in each of the skin-stretching parts, the surface area of the tip surface is 1 × 10⁻³ mm² or more and the height from the front surface of the sheet-shaped base material to the tip surface is 30 µm or more and 300 µm or less, and the skin-stretching parts are disposed spaced apart such that a spacing between boundaries of mutually adjacent skin-stretching parts and the sheet-shaped base material is greater than or equal to a value calculated by dividing the square of the height of the mutually adjacent skin-stretching part by 100.

6. The microneedle sheet according to claim 5, wherein
in the skin-stretching part, a length from a root of the needlelike-projection part to an end part of the tip surface is 8 µm or more and 20 µm or less.

7. The microneedle sheet according to any one of claim 1 to claim 6, wherein
the plurality of microneedles is disposed with a density wherein the surface area occupied by the tip surfaces per unit of area is 0.3% or more.

8. A transdermal patch comprising
the microneedle sheet according to any one of claim 1 to claim 7; and
a pressing part configured to maintain a state in which the microneedle sheet is pressed against the skin.
